**Europäisches Patentamt**

**European Patent Office**

**Office européen des brevets**

(19)

(11) Veröffentlichungsnummer: **0 329 962 B1**

## EUROPÄISCHE PATENTSCHRIFT

(45) Veröffentlichungstag der Patentschrift: **01.06.94**

(21) Anmeldenummer: **89101223.9**

(22) Anmeldetag: **25.01.89**

(51) Int. Cl.5: **B01D 53/22**, C07C 7/144, B01D 53/04

(54) **Verfahren zum Austrag organischer Verbindungen aus Luft/Permanentgasgemischen.**

(30) Priorität: **26.02.88 DE 3806107**

(43) Veröffentlichungstag der Anmeldung: **30.08.89 Patentblatt 89/35**

(45) Bekanntmachung des Hinweises auf die Patenterteilung: **01.06.94 Patentblatt 94/22**

(84) Benannte Vertragsstaaten: **AT BE CH DE ES FR GB IT LI NL SE**

(56) Entgegenhaltungen: **EP-A- 0 247 585**

(73) Patentinhaber: **GKSS-FORSCHUNGSZENTRUM GEESTHACHT GMBH Max-Planck-Strasse D-21502 Geesthacht(DE)**

(72) Erfinder: **Behling, Dieter, Dr. Bramweg 14 D-2000 Hamburg 55(DE)** Erfinder: **Hattenbach, Karl, Dr. Schulstrasse 2 c D-2054 Geesthacht(DE)** Erfinder: **Ohlrogge, Klaus Am Gehölz 5**

**D-2054 Geesthacht(DE)** Erfinder: **Peinemann, Klaus-Victor, Dr. Husumer Strasse 11 D-2057 Reinbek(DE)** Erfinder: **Wind, Jan Reggenhof 26 D-2000 Barsbüttel(DE)**

(74) Vertreter: **Niedmers, Ole, Dipl.-Phys. Patentanwälte Niedmers & Schöning Stahltwiete 23 D-22761 Hamburg (DE)**

**Beschreibung**

Die Erfindung betrifft ein Verfahren zum Austrag organischer Verbindungen aus Luft/Permanentgasgemischen, bei dem das Luft/Permanentgasgemisch (Rohmedium) auf eine erste Gastrennmembran geleitet und in einen mit organischen Verbindungen angereicherten (Permeat) und in einen abgereicherten (Retentat) Gasstrom aufgetrennt wird, wobei permeatseitig ein unteratmosphärischer Druck erzeugt wird, und aus dem über eine Rückgewinnungseinrichtung geleiteten angereicherten Gasstrom die organischen Verbindungen zurückgewonnen werden, und der die Gastrennmembraneinrichtung verlassende, abgereicherte Gasstrom an die Umgebung abgegeben wird, und daß ein aus der Rückgewinnungseinrichtung austretender Gasstrom erneut dem Luft/Permanentgasgemisch zugeführt wird, nachdem dieses druckerhöht wurde.

Ein Verfahren dieser Art ist bekannt (EP-A-0 247 585). Bei diesem bekannten Verfahren wird das zu trennende Gasgemisch auf einen Unterdruckförderer (blower) und von dort vor Eintritt in eine Membrantrenneinrichtung auf einen Filter gegeben. Der Unterdruckförderer dient dem Gastransport und erzeugt einen geringfügigen Überdruck von 40-60 mbar, um im nachfolgenden Abscheider für Aerosole oder dergleichen zwangsweise auftretende Druckminderungen von 20 mbar ausgleichen zu können. Das zu trennende Gasgemisch wird somit nur geringfügig über Normaldruck liegend der Membrantrenneinrichtung zugeführt.

Allgemein gilt, daß der Gastransport durch eine Gastrennmembran proportional der transmembranen Partialdruckdifferenz ist. Das Trennvermögen einer Gastrennmembran und damit die Reinheit des erzeugten Permeats hängt im wesentlichen vom Verhältnis des Drucks auf der Membranvorderseite zum Permeatdruck, d.h. vom Verhältnis des Retentatdrucks zum Permeatdruck ab. Eine Gastrennmembran bzw. ein Verfahren unter Verwendung einer derartigen Gastrennmembran arbeitet optimal nur dann, wenn sich sowohl ein hoher Vordruck als auch ein der Selektivität der Gastrennmembran angepaßtes Druckverhältnis einstellen läßt.

Der Nachteil eines anderen bekannten Verfahrens (US-A-4 553 983) liegt im wesentlichen darin, daß diesen Kriterien nur bei verhältnismäßig niedrigen Konzentrationen organischer Verbindungen im Luft/Permanentgasgemisch Rechnung getragen werden kann, wobei es bei diesem bekannten Verfahren besonders nachteilig ist, daß die gemäß diesem bekannten Verfahren abgegebene Abluft noch eine hohe Konzentration an organischen Verbindungen enthält. Das bekannte Verfahren kann beispielsweise die vom Gesetzgeber in der Bundesrepublik Deutschland vorgegebene einschlägige Vorschrift für das Höchstmaß an organischen Verbindungen in Abluft von Industrieanlagen (TA-Luft) nicht einhalten.

Es besteht andererseits ein sehr großer Bedarf ein Verfahren bereitzustellen, mit dem beispielsweise im Zuge der immer knapper werdenden Rohstoffresourcen einerseits und dem gestiegenen Umweltbewußtsein andererseits aus den beträchtlichen Mengen von luft/gasförmigen Erdölderivaten, die sich in Mineralöltanks bilden, den Anteil der organischen Verbindungen daraus wiederum zu verflüssigen und einer Nutzung zuzuführen und nicht an die Umwelt zu entlassen.

Es ist Aufgabe der vorliegenden Erfindung, ein Verfahren zu schaffen, mit dem bei niedrigem Eigenenergiebedarf ein Optimum an organischen Verbindungen aus einem Luft/Permanentgasgemisch ausgetragen wird, bei dem das an die Umgebung abgegebene Gasgemisch einen derart geringen Anteil an organischen Verbindungen enthält, daß die in der Bundesrepublik Deutschland durch die TA-Luft vorgegebenen Werte in bezug auf den Fremdgasanteil eingehalten werden, und das apparativ wenig Aufwand erfordert und auch bei hohen Konzentrationen organischer Verbindungen im Luft/Permanentgasgemisch (Rohmedium) ohne Schwierigkeiten funktioniert.

Gelöst wird die Aufgabe gemäß der Erfindung dadurch, daß der Druck des primären Luft/Permanentgasgemisches vor Eintritt in die erste Gastrennmembraneinrichtung durch Verdichtung weit über Normaldruck erhöht wird und der Druck des angereicherten Gasstroms nach Austritt aus der ersten Gastrennmembraneinrichtung zur Schaffung eines großen Druckverhältnisses weit unter den Druck des verdichteten Luft/Permanentgasgemisches erniedrigt wird, und der Druck des angereicherten Gasstroms vor Eintritt in die Rückgewinnungseinrichtung durch Verdichtung wiederum erhöht wird.

Der Vorteil des vorgeschlagenen Verfahrens besteht im wesentlichen darin, daß einerseits hohe Konzentrationen von organischen Verbindungen im Luft/Permanentgasgemisch ausgetragen werden können, da das für einen optimalen Trenneffekt wichtige Druckgefälle zwischen Membranoberseite und Unterseite erfindungsgemäß eingestellt wird, indem der Druck des primären Luft/Permanentgasgemisches vor Eintritt in die Gastrennmembraneinrichtung erhöht wird und der Druck des angereicherten Gasstroms nach Austritt aus der Gastrennmembraneinrichtung auf vorbestimmte Weise erniedrigt wird. Dadurch, daß der aus der Gastrennmembraneinrichtung austretende druckerniedrigte angereicherte Gasstrom vor Eintritt in die Rückgewinnungseinrichtung druckerhöht wird, kann die Rückgewinnungseinrichtung effektiver arbeiten.

Gemäß einer vorteilhaften Ausführungsform der Erfindung wird die die Rückgewinnungseinrichtung verlassende Abluft dem primären Luft/Permanentgasgemisch unmittelbar vor Eintritt in die Gasmembraneinrichtung wiederum zugeführt, so daß ein in der Abluft verbleibender Restanteil an organischen Verbindungen wiederum der Gastrennmembraneinrichtung zugeführt werden kann und nicht, wie beispielsweise beim bekannten Verfahren, an die Umgebung abgegeben wird.

Vorteilhafterweise wird dabei die Abluft nach Austritt aus der Rückgewinnungseinrichtung in ihrem Druck vermindert, d.h. so entspannt, daß sie sich im wesentlichen auf gleichem Druck wie das unmittelbar in die Gastrennmembraneinrichtung eintretende Luft/ Permanentgasgemisch befindet.

Nach einer anderen Ausführungsform des Verfahrens wird die die Rückgewinnungseinrichtung verlassende Abluft auf eine zweite Gastrennmembraneinrichtung gegeben, deren abgereicherter Gasstrom an die Umgebung abgegeben wird und deren angereicherter Gasstrom unmittelbar mit dem die erste Gastrennmembraneinrichtung verlassenden angereicherten Gasstrom zusammengeführt wird. Vorzugsweise ist dabei zur Einstellung des Betriebsdrucks der zweiten Gastrennmembraneinrichtung der Druck des diese verlassenden Gasstroms einstellbar. Diese Ausgestaltung des Verfahrens gestattet mit einfachen Mitteln eine nochmalige Reinigung der aus der Rückgewinnungseinrichtung austretenden Abluft, d.h. Aufspaltung in ein angereichertes Gasgemisch und Rückführung zur Rückgewinnungseinrichtung und eine verbesserte Reinigung des als Retentat austretenden Gasgemisches auf eine gewünschte, an die Umgebung abzugebende Abluftkonzentration.

Bei einer weiteren anderen Ausführungsform der Erfindung wird das druckerhöhte primäre Luft/Permanentgasgemisch auf eine dritte Gastrennmembraneinrichtung gegeben, deren sauerstoffabgereicherter Gasstrom in die erste Gastrennmembraneinrichtung eintritt und deren sauerstoffangereicherter Gasstrom an die Umgebung abgegeben wird, wobei die dritte Gastrennmembraneinrichtung erhöhte sauerstoffpassierende Eigenschaften aufweist. In dieser dritten Gastrennmembraneinrichtung wird beispielsweise ein Sauerstoff-Stickstoffverhältnis, wenn diese Gastrennmembraneinrichtung eine spezielle Sauerstoff-Stickstoff-Selektivität aufweist, auf der Permeatseite zugunsten des Sauerstoffs verschoben, so daß in die erste ursprüngliche Gastrennmembraneinrichtung schon ein sauerstoffabgereicherter Gasstrom eintritt, d.h. es wird ein angereichertes Permeat auf die erste Gastrennmembraneinrichtung gegeben.

Bei einer noch anderen weiteren Ausführungsform des Verfahrens wird der angereicherte Gasstrom vor Eintritt in die Rückgewinnungseinrichtung auf eine dritte Gastrennmembraneinrichtung gegeben, deren sauerstoffabgereichter Gasstrom auf die Rückgewinnungseinrichtung und deren sauerstoffangereicherter Gasstrom an die Umgebung gegeben wird, wobei die dritte Gastrennmembraneinrichtung erhöhte sauerstoffpassierende Eigenschaften aufweist. Diese Ausführungsform wird insbesondere zur Vermeidung einer Aufkonzentration des Sauerstoffs im Rückgewinnungskreislauf eingesetzt werden. Dieser Schritt ist insbesondere dann wirtschaftlich, wenn die Rückgewinnungseinrichtung dabei unter Druck betrieben wird, d.h. die Rückgewinnung unter Druck erfolgt und das Druckinventar für beide Gastrennmembraneinrichtungen genutzt wird.

Die zuvor beschriebene vorteilhafte Ausbildung einer weiteren Ausführungsform des Verfahrens kann noch dadurch vorteilhafterweise modifiziert werden, daß die aus der Rückgewinnungseinrichtung austretende Abluft auf eine dritte Gastrennmembraneinrichtung gegeben wird, deren sauerstoffabgereicherter Gasstrom auf die zweite Gastrennmembraneinrichtung gegeben wird und deren sauerstoffangereicherter Gasstrom an die Umgebung gegeben wird, wobei die dritte Gastrennmembraneinrichtung erhöhte sauerstoffpassierende Eigenschaften aufweist. Auch diese Ausgestaltung des Verfahrens wird zur Vermeidung einer Aufkonzentrierung des Sauerstoffs eingesetzt, so daß das Verfahren auch in diesem Falle besonders wirtschaftlich betrieben werden kann.

Gemäß einer letzten Ausführungsform eines vorteilhaften Verfahrens wird der aus der zweiten Gastrennmembraneinrichtung austretende abgereicherte Gasstrom vor Abgabe an die Umgebung zum Antrieb einer Pumpeneinrichtung genutzt, wobei der sauerstoffangereicherte Gasstrom der dritten Gastrennmembraneinrichtung auf die Saugseite der Pumpeinrichtung zur Erzeugung eines Unterdrucks des Gasstroms gegeben wird. Auch auf diese Weise kann vorteilhafterweise der Betriebsdruck der zweiten Gastrennmembraneinrichtung anstelle eines Druckreglers über den aus ihr austretenden abgereicherten Gasstrom eingestellt werden und der Druck für den Betrieb der dritten Gastrennmembraneinrichtung dadurch wiederum zur Abtrennung organischer Verbindungen mit Hilfe der Pumpeneinrichtung zur Erzeugung eines Unterdrucks auf der sauerstoffangereicherten Seite (Permeatseite der Sauerstofftrennmembran) der dritten Gastrennmembraneinrichtung genutzt werden.

Ebenfalls kann bei allen vorgenannten vorteilhaften Verfahren der abgereicherte Gasstrom nach Austritt aus der ersten Gastrennmembraneinrichtung in seinen Druck vermindert werden, bevor er an die Umgebung abgegeben werden kann. Die Einstellung des Drucks des Retentats ermöglicht eine unmittelbare

EP 0 329 962 B1

Anpassung des Druckverhältnisses entsprechend der Selektivität der Gastrennmembraneinrichtung.

Die Rückgewinnung der organischen Verbindungen aus dem angereicherten Gasstrom in der Rückgewinnungseinrichtung kann vorteilhafterweise durch Wärmeentzug, Druck oder auch durch Sorption erfolgen. Vorteilhafterweise können auch beliebige Kombinationen der vorgenannten Arten der Rückgewinnung der organischen Verbindungen in einer Rückgewinnungseinrichtung zur Anwendung kommen. Die Sorption kann beispielsweise eine physikalische Absorption, eine chemische Absorption oder auch eine Adsorption sein.

Die eigentliche Gastrennmembran kann vorzugsweise eine Polyetherimid-Komposit-Membran sein, wobei dieses vorzugsweise für die erste und zweite Gastrennmembraneinrichtung gilt.

Für die dritte Gastrennmembraneinrichtung wird vorzugsweise eine asymmetrische Polyetherimidmembran Verwendung finden, da diese eine hohe Sauerstoff-Kohlenwasserstoffselektivität aufweist. Schließlich ist es vorteilhaft, daß bei gewissen Ausgestaltungen des Verfahrens das zu trennende Luft/Permanentgasgemisch vor Druckerhöhung inertisiert wird, so daß weitgehend verhindert wird, daß sich infolge der Druckerhöhung ein explosives Gemisch bildet.

Die Erfindung wird nun unter Bezugnahme auf die nachfolgenden schematischen Zeichnungen anhand mehrerer Ausführungsbeispiele beschrieben. Darin zeigen:

Fig. 1    eine Anordnung, mit der das erfindungsgemäße Verfahren gemäß einer ersten Ausführungsform ausgeführt wird,

Fig. 2    eine Anordnung zur Ausführung des Verfahrens, gemäß einer zweiten Ausführungsform,

Fig. 3    eine Anordnung zur Ausführung des Verfahrens gemäß einer dritten Ausführungsform,

Fig. 4    eine Anordnung zur Ausführung des Verfahrens gemäß einer vierten Ausführungsform,

Fig. 5    eine Anordnung zur Ausführung des Verfahrens gemäß einer fünften Ausführungsform,

Fig. 6    eine Darstellung der Permeabilität einer Membran für verschiedene Rohmediumskomponenten in Abhängigkeit von der Temperatur,

Fig. 7    eine Darstellung der Permeabilität einer Membran für n-Butan in Abhängigkeit vom Druck bei verschiedenen Temperaturen,

Fig. 8    eine Darstellung der Permeabilität einer Membran für Propan in Abhängigkeit des Druckes bei verschiedenen Temperaturen und

Fig. 9    eine Darstellung des Gasflusses durch zwei unterschiedliche Gastrennmembraneinrichtungen zur Erläuterung von Tabelle 4.

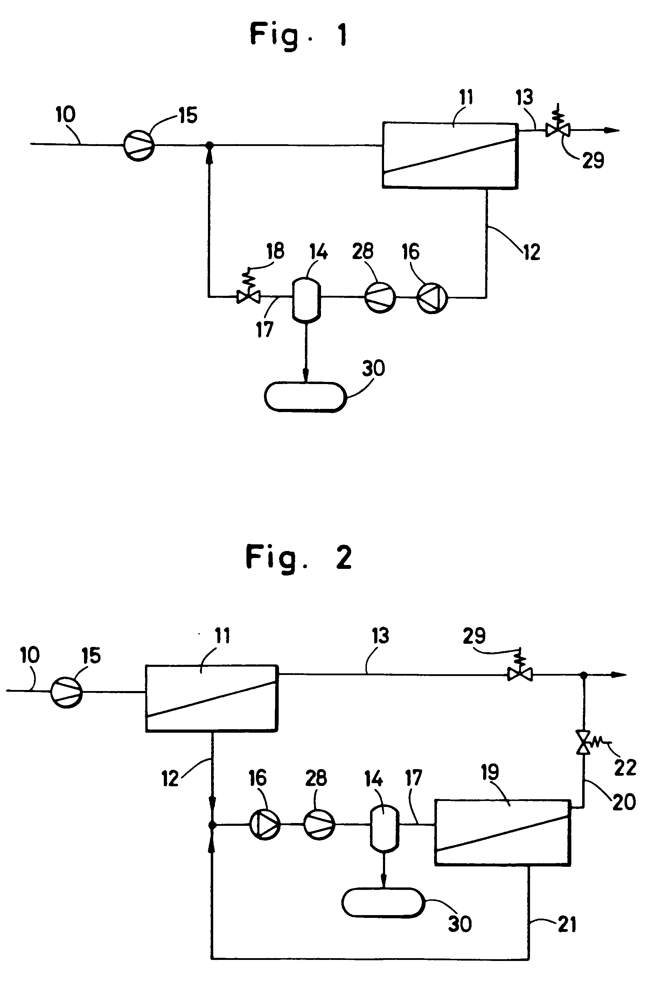

Eine erste Anordnung zur Ausführung des erfindungsgemäßen Verfahrens ist in Fig. 1 dargestellt und wird im Folgenden beschrieben. Über eine Rohgasleitung 10 wird ein mit organischen Verbindungen belastetes Luft/Permanentgasgemisch (Rohmedium) einem Verdichter 15 zugeführt. In einer ersten Gastrennmembraneinrichtung 11 wird das Rohmedium in einen mit organischen Verbindungen abgereicherten Gasstrom 13 (Retentat) und einen mit organischen Verbindungen angereicherten Gasstrom 12 (Permeat) aufgeteilt. Das Permeat 12 wird über eine Vakuumpumpe 16 und von dort über einen Verdichter 28 einer Rückgewinnungseinrichtung 14 zugeführt, in der durch Druck, Wärmeentzug oder Sorption oder durch Kombination dieser Austragmechanismen die organischen Verbindungen im Permeat 12 verflüssigt und einem Vorratsbehälter 30 zugeführt werden. Die Abluft 17 von der Rückgewinnungseinrichtung 14 wird mit Hilfe eines Druckreglers 18 auf den Druck des Verdichters 15 entspannt und dem druckerhöhten Luft/Permanentgasgemisch 10 (Rohmedium) beigemischt. Der Durchfluß durch die Gastrennmembraneinrichtung 11 wird mit Hilfe eines Druckreglers 29 eingestellt.

Bei der in Fig. 2 dargestellten Anordnung zur Ausführung des Verfahrens wird ebenfalls über eine Rohgasleitung ein mit organischen Verbindungen belastetes Luft/Permanentgasgemisch 10 (Rohmedium) einem Verdichter 15 zugeführt. In der Gastrennmembraneinrichtung 11 wird das Rohmedium 10 in einen mit organischen Verbindungen abgereicherten Gasstrom 13 (Retentat) und einen mit organischen Verbindungen angereicherten Gasstrom 12 (Permeat) aufgeteilt. Das Permeat 12 wird über eine Vakuumpumpe 16 und einen Verdichter 28 einer Rückgewinnungseinrichtung 14 zugeführt. Der Rückgewinnungseinrichtung ist eine zweite Gastrennmembraneinrichtung 19 nachgeschaltet. Unter Ausnutzung des erhöhten Vordrucks wird die die Rückgewinnungseinrichtung 14 verlassende Abluft 17 in einen abgereicherten Gasstrom 20 (Retentat) mit niedrigem Anteil organischer Verbindungen und einen angereicherten Gasstrom 21 (Permeat) aufgespalten. Der angereicherte Gasstrom 21 (Permeat) wird dem Permeat 12 der ersten Gastrennmembraneinrichtung 11 beigemischt. Der Betriebsdruck der zweiten Gastrennmembraneinrichtung 19 wird über einen Druckregler eingestellt. Der abgereicherte Gasstrom 20 (Retentat), der praktisch die Abluft der zweiten Gastrennmembraneinrichtung 19 bildet, wird dem Retentat 13 bzw. der Abluft 13 der ersten Gastrennmembraneinrichtung beigemischt. Die in der Rückgewinnungseinrichtung 14 gewonnenen flüssigen organischen Verbindungen, die grundsätzlich auf gleiche Weise wie bei der Anordnung gemäß Fig. 1 gewonnen werden können, werden in den Vorratsbehälter 30 geleitet. Auch bei dieser Ausgestaltung des Verfahrens wird die Durchflußmenge des Luft/Permanentgasgemisches 10 (Rohmedium) durch die erste

4

Gastrennmembraneinrichtung 11 mit Hilfe des Druckreglers 29 eingestellt.

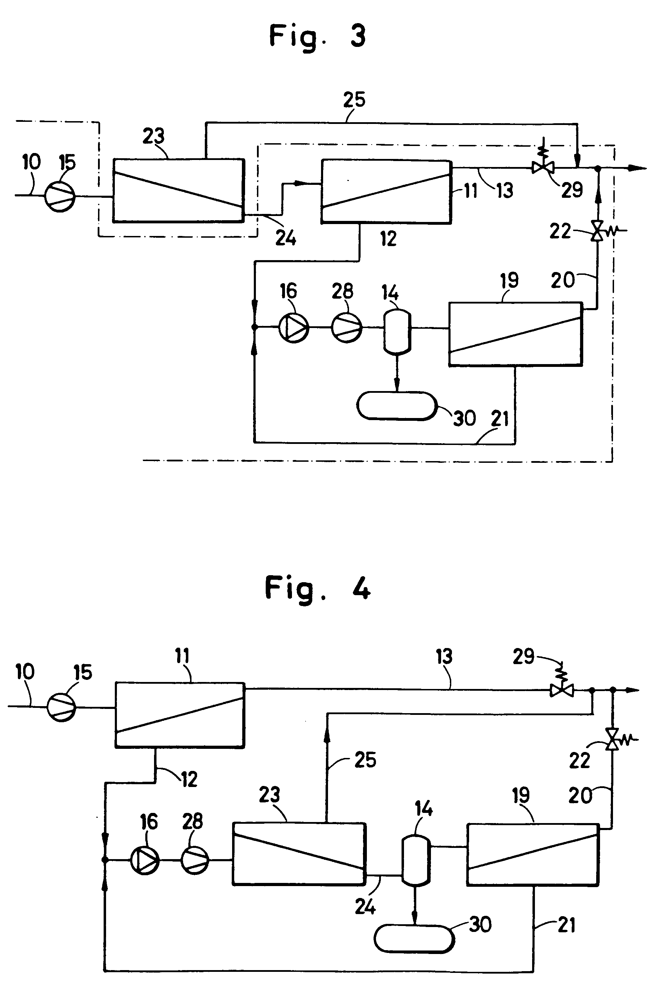

Bei der in Fig. 3 dargestellten Anordnung zur Ausführung des Verfahrens entspricht der durch die strichpunktierte Linie eingefaßte Aufbau der Anordnung der im Zusammenhang mit Fig. 2 beschriebenen Anordnung, so daß diesbezüglich auf die Beschreibung der Anordnung von Fig. 2 verwiesen wird. In den Strom des Luft/Permanentgasgemisches 10 (Rohmedium) ist zwischen dem Verdichter 15 und der ersten Gastrennmembraneinrichtung 11 eine dritte Gastrennmembran 23 angeordnet, die bevorzugt Sauerstoff passieren läßt, so daß ein Teil des Sauerstoffs aus dem Luft/Permanentgasgemisch 10 (Rohmedium) als sauerstoffangereicherter Gasstrom 25 (Sauerstoffpermeat) abgetrennt wird. Der sauerstoffangereicherte Gasstrom 25 wird dem organisch abgereicherten Gasstrom 13 der ersten Gastrennmembraneinrichtung 11 beigemischt. Der sauerstoffabgereicherte Gasstrom 24 der dritten Gastrennmembraneinrichtung 23 wird, wie im Zusammenhang mit Fig. 2 beschrieben, als (sauerstoffabgereichertes) Luft/ Permanentgasgemisch der ersten Gastrennmembraneinrichtung 11 zugeführt.

Die in Fig. 4 dargestellte modifizierte Ausgestaltung einer Anordnung zur Ausführung des Verfahrens ist grundsätzlich wie die im Zusammenhang mit Fig. 2 beschriebene Anordnung aufgebaut. Insofern wird auf die Beschreibung der Anordnung von Fig. 2 verwiesen. Unterschiedlich gegenüber der in Fig. 2 dargestellten Anordnung ist jedoch, daß zwischen dem Verdichter 28 und der Rückgewinnungseinrichtung 14 eine dritte Gastrennmembraneinrichtung 23 eingefügt ist. Die dritte Gastrennmembraneinrichtung 23 dient auch in diesem Falle zur Sauerstoffabreicherung der aus der ersten Gastrennmembraneinrichtung 13 und aus der zweiten Gastrennmembraneinrichtung 19 kommenden, mit organischen Verbindungen angereicherten Gasströme 12 und 21. Der sauerstoffangereicherte Gasstrom 25 der dritten Gastrennmembraneinrichtung 23 wird dem von organisch abgereicherten Gasstrom 13 (Retentat) der ersten Gastrennmembraneinrichtung 11 beigemischt. Der sauerstoffabgereicherte Gasstrom 24 (Sauerstoffretentat) der Gastrennmembraneinrichtung 23 wird der Rückgewinnungseinrichtung 14 zugeführt, wie es im Zusammenhang mit der Darstellung der Anordnung von Fig. 2 beschrieben wurde. Die in Fig. 4 dargestellte Anordnung kann auch noch dadurch modifiziert werden, daß die dritte Gastrennmembraneinrichtung 23 zwischen die Rückgewinnungseinrichtung 14 und die zweite Gastennmembraneinrichtung 19 geschaltet ist.

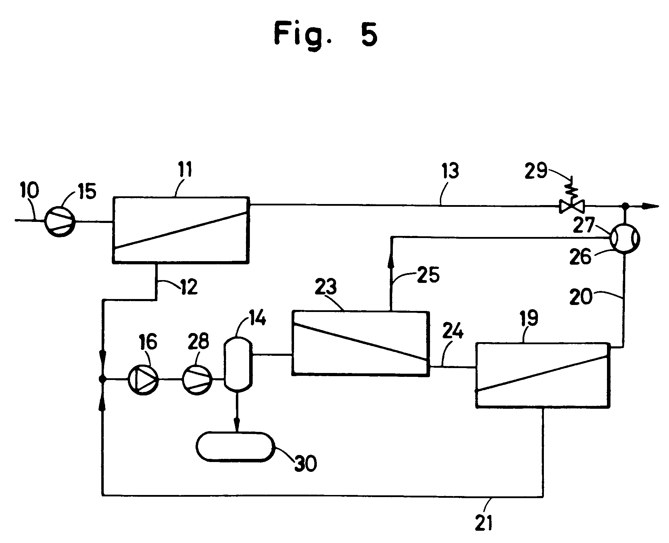

Die in Fig. 5 dargestellte Anordnung zur Ausführung des Verfahrens entspricht der im Zusammenhang mit Fig. 4 (modifizierte Form) beschriebenen Anordnung, wobei bezüglich der Funktion auf die Beschreibung von Fig. 4 verwiesen wird. Gegenüber dem Aufbau der Anordnung von Fig. 4 ist lediglich unterschiedlich, daß anstelle des Druckreglers 22 im abgereicherten Gasstrom 20 der zweiten Gastrennmembran 19 eine Pumpeneinrichtung 26 vorgesehen ist, wobei die Pumpeneinrichtung 26 eine Strahlpumpe sein kann, für die der Druck des abgereicherten Gasstroms 20 (Retentatdruck) benutzt wird. Der sauerstoffangereicherte Gasstrom 25 (Sauerstoffpermeat) der dritten Gastrennmembraneinrichtung 23 wird der Saugkammer der Pumpeneinrichtung 26 zugeführt, mit der Folge, daß ein Unterdruck auf der Seite des sauerstoffangereicherten Gasstroms 25 (Sauerstoffpermeat) der dritten Gastrennmembran 23 erzeugt wird.

Grundsätzlich können zur Ausführung des erfindungsgemäßen Verfahrens gemäß den vorangehend beschriebenen unterschiedlichen Anordnungen beliebige geeignete Pumpeneinrichtungen und Verdichtereinrichtungen verwendet werden. Vorteilhafterweise werden jedoch die Verdichter 15 und 28 durch Flüssigkeitsringpumpen gebildet, wobei die Betriebsflüssigkeit dieser Pumpen eine organische Verbindungen sein können, durch die ein Teil der kondensierenden Komponenten durch Absorption aufgenommen werden kann.

Die erste Gastrennmembraneinrichtung 11 und die zweite Gastrennmembraneinrichtung 23 verwenden vorzugsweise als Membran eine Polyetherimid-Komposit-Membran, die bevorzugt organische Verbindungen durchläßt. Die dritte Gastrennmembraneinrichtung 23 verwendet bevorzugt als Membran eine integral asymmetrische Polyetherimidmembran, die bevorzugt für Sauerstoff durchlässig ist.

Wie schon im Zusammenhang mit der in Fig. 1 beschriebenen Anordnung angedeutet, kann die Rückgewinnungseinrichtung 14 zum Austrag der organischen Verbindungen aus dem organisch angereicherten Gasstrom unter Ausnutzung verschiedener physikalischer Prozesse betrieben werden, beispielsweise durch Druck, Wärmeentzug und Sorption oder aus Kombinationen dieser Prozesse. Die Sorption selbst kann wiederum eine physikalische Absorption, eine chemische Absorption sein oder durch Adsorption durchgeführt werden. Auch der Wärmeentzug kann entweder direkt oder indirekt erfolgen.

Die im Zusammenhang mit Fig. 2 beschriebene Anordnung zur Ausführung des erfindungsgemäßen Verfahrens wurde mittels einer Modellrechnung untersucht. Es zeigte sich dabei eine Einsparung an Membranfläche und eine höhere Abluftreinheit. Als Beispiel wurde dabei eine Anlage zur Rückgewinnung von Benzinkomponenten aus der Abluft von Tanklagern für Otto-Kraftstoffe unter Berücksichtigung der Auswurfbegrenzungen organischer Verbindungen gewählt. Zur Rückgewinnung der Benzinkomponenten wurde eine Druckkondensation bei 10 bar angenommen.

5

Tabelle 1 (Permeabilitäten)

Gaspermeabilitätskoeffizienten von Polydimethylsiloxan (25°C)
in $10^{-6} \cdot (m^3 \cdot m)/(m^2 \cdot h \cdot bar)$

| Sauerstoff | Stickstoff | Propan | n-Butan | n-Pentan |
|---|---|---|---|---|
| 1,62 | 0,76 | 11,07 | 24,30 | 54,00 |

* General Electric Broschüre, März 1982, "Permselective
Membranes"

Diese Werte wurden für Polyetherimid/Silicon Kompositmembranen bestätigt, wobei auch Temperatur-
und Druckabhängigkeiten der Benzinkomponente gemessen wurden.

Die Tabelle 2 zeigt Berechnungen unter Zugrundelegung des Aufbaus der erfindungsgemäßen Anordnung zur Ausführung des Verfahrens gemäß Fig. 1, Tabelle 3 zeigt Berechnungen unter Zugrundelegung
des Aufbaus der Anordnung zur Ausführung es erfindungsgemäßen Verfahrens gemäß Fig. 2.

Tabelle 4  Beispiel einer Membran zur  Sauerstoffabtrennung.
Membrantyp: integral asymmetrische Polyetherimidmembran

| Gasflüsse (25°C) $Nm^3/m^2 \cdot h \cdot bar$ | $N_2$ | $O_2$ | Methan | Ethan |
|---|---|---|---|---|
| | 0,00117 | 0,00932 | 0,00081 | 0,00051 |

| Propan | Butan |
|---|---|
| 0,00048 | 0,00031 |

| Selektivitäten | $O_2/C_1$ | $O_2/C_2$ | $O_2/C_3$ | $O_2/C_4$ |
|---|---|---|---|---|
| | 11,5 | 18,3 | 19,4 | 30,1 |

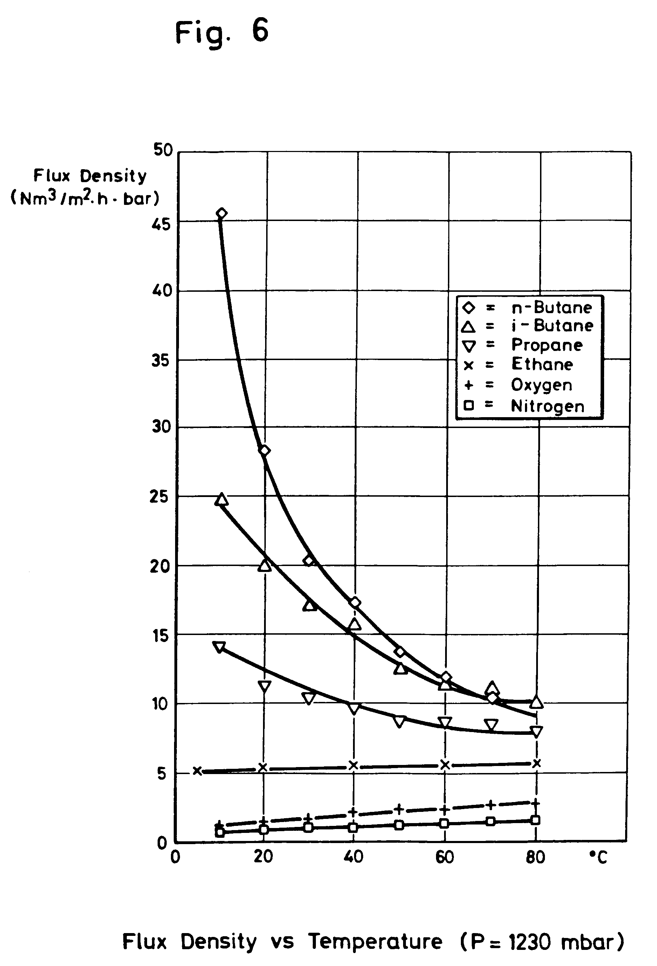

Fig. 6, 7 und 8 zeigen für einige Hauptkomponenten aus den Benzindampfluftgemischen das Permeationsverhalten in Abhängigkeit von Druck und Temperatur.

In Fig. 6 sind Reingasmessungen bei unterschiedlichen Temperaturen und gleichem Vordruck dargestellt. Hier zeigt sich eine starke Abhängigkeit der Flußdichte von der Temperatur. Während für Sauerstoff
und Stickstoff die Flußdichte mit steigender Temperatur zunimmt, geht die Flußdichte für n-Butan, i-Butan
und Propan stark zurück. Diese Darstellung zeigt, daß die Arbeitstemperatur für eine Membrantrenneinrichtung zwischen 20°C und 40°C liegen sollte, damit mit Membranen noch eine ausreichende Trennleistung
erzielt werden kann.

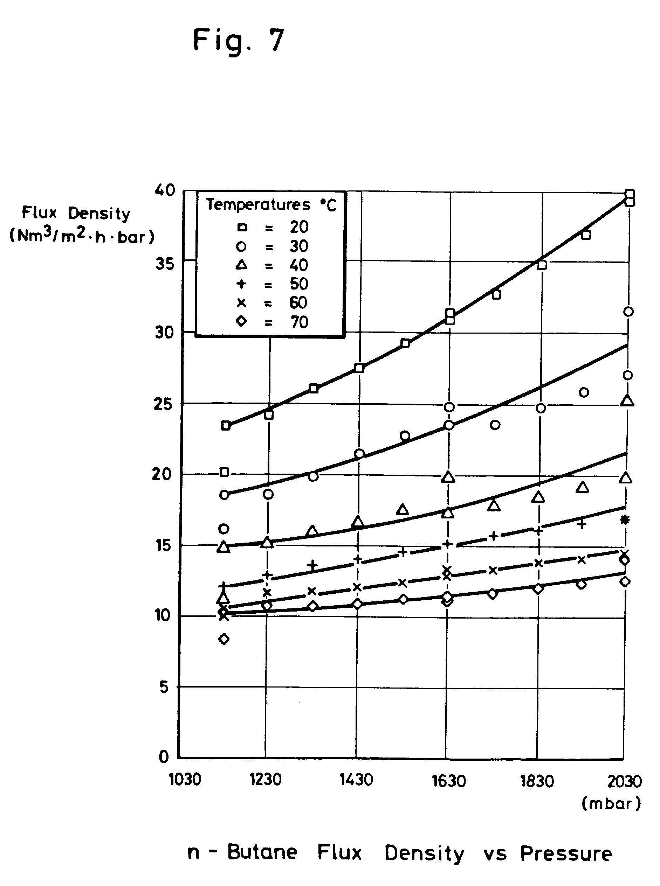

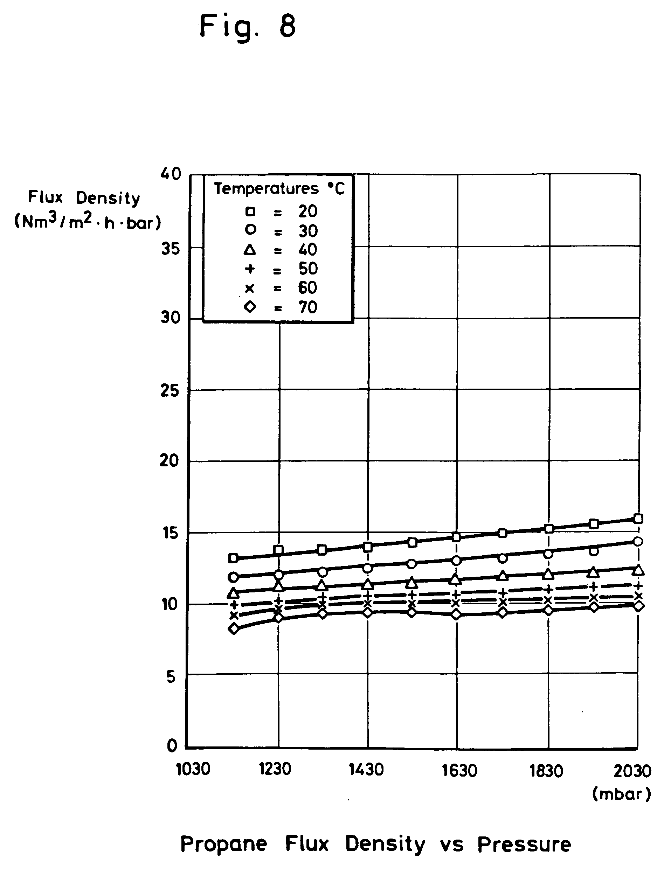

Fig. 7 und 8 zeigen die Abhängigkeit der Flußdichte von n-Butan und Propan vom Vordruck bei
bestimmten Temperaturen. Aus den Darstellungen ist zu erkennen, daß die Flußdichte von Propan und
Butan mit fehlendem Druck abnimmt. D.h. je höher der Partialdruck der Komponenten n-Butan und Propan ist, desto höher ist die Flußdichte. Dieses Verhalten wurde auch für andere Hauptkomponeneten wie i-Butan, i-Pentan und n-Pentan gemessen.

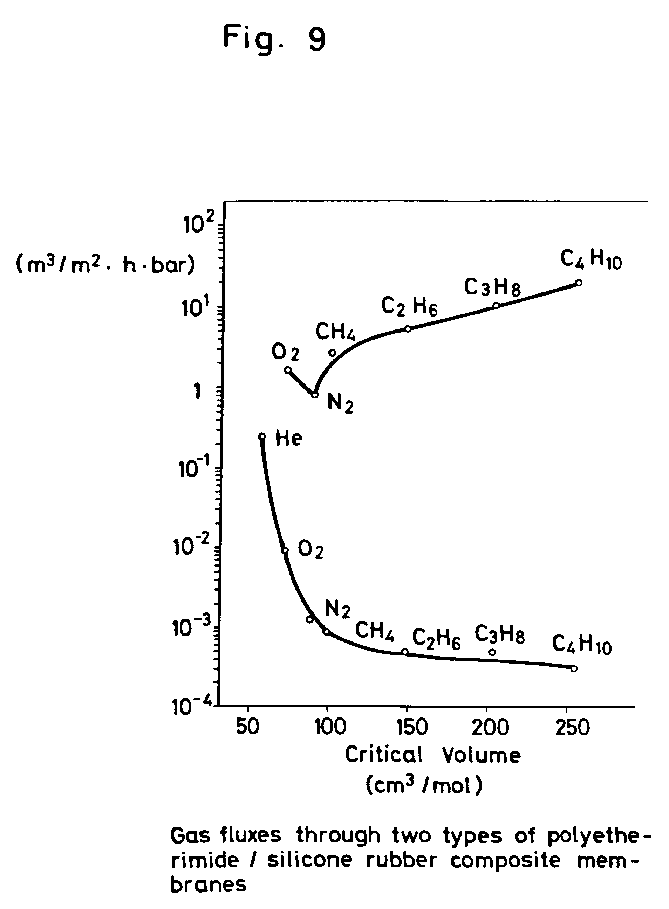

Fig. 9 dient zur Erläuterung von Tabelle 4. Hier ist der Gasfluß durch zwei Membrantypen, einer integral asymmetrischen Polyetherimidmembran und einer Silikonkompositmembran, dargestellt. Die obere Kurve zeigt das Permeationsverhalten der Silikonkompositmembran mit hohen Kohlwasserstoffflüssen und niedrigen Sauerstoff- und Stickstoffflüssen. Die untere Kurve zeigt Meßwerte für die integral asymmetrische Polyetherimidmembran, die einen hohen Sauerstofffluß und niedrige Kohlenwasserstoffflüsse aufweist.

```
FLUESSE L0         EXPONENTEN        DAMPFDRUECKE

IN cbm/qm*h*bar    IN 1/bar          IN bar

Propan          :   11.0000   0.1700   10.9040
i-Butan         :    9.5000   0.6700    3.7600
n-Butan         :    9.9000   0.6700    2.8000
i-Pentan        :   21.0000   0.8100    1.1000
n-Pentan        :   21.0000   0.8100    0.8230
Hexan - Oktan   :   20.0000   0.0000    0.2520
Benzol/Toluol   :   20.0000   0.0000    0.1610
Sauerstoff      :    1.3600   0.0000
Stickstoff      :    0.6400   0.0000
                :    0.0000   0.0000
```

2 bar / 52% KV / 10 bar Kon.

```
SCHRITTFLAECHE      :    2.0000 qm

MEMBRANFLAECHE   : 120.0000 qm    PERMEATDRUCK   :   0.2000 bar
                                  DRUCKVERHAELTNIS :  10.00
                                  KONDENSATDRUCK :  10.0000 bar

ZULAUF           :   300.00 cbm/h
ZUSAMMENSETZUNG  :     3.40 % Propan          6.00 % i-Butan
                      28.50 % n-Butan         4.90 % i-Pentan
                       3.90 % n-Pentan        4.80 % Hexan - Oktan
                       0.60 % Benzol/Toluol  10.10 : Sauerstoff
                      37.80 % Stickstoff      0.00 %

ZULAUF (GEMISCH) :   535.23 cbm/h
ZUSAMMENSETZUNG  :     5.01 % Propan          5.26 % i-Butan
                      22.69 % n-Butan         3.20 % i-Pentan
                       2.46 % n-Pentan        2.79 % Hexan - Oktan
                       0.34 % Benzol/Toluol  18.47 : Sauerstoff
                      39.76 % Stickstoff      0.00 %

PERMEAT          :   390.41 cbm/h
ZUSAMMENSETZUNG  :     6.84 % Propan          7.17 % i-Butan
                      30.92 % n-Butan         4.39 % i-Pentan
                       3.37 % n-Pentan        3.83 % Hexan - Oktan
                       0.47 % Benzol/Toluol  17.57 : Sauerstoff
                      25.46 % Stickstoff      0.00 %

PERMEAT NACH
KONDENSATION     :   235.23 cbm/h
ZUSAMMENSETZUNG  :     7.07 % Propan          4.32 % i-Butan
                      15.29 % n-Butan         1.04 % i-Pentan
                       0.62 % n-Pentan        0.23 % Hexan - Oktan
                       0.02 % Benzol/Toluol  29.15 : Sauerstoff
                      42.25 % Stickstoff      0.00 %

RETENTAT         :   144.80 cbm/h
ZUSAMMENSETZUNG  :     0.09 % Propan          0.12 % i-Butan
                       0.53 % n-Butan         0.01 % i-Pentan
                       0.01 % n-Pentan        0.01 % Hexan - Oktan
                       0.00 % Benzol/Toluol  20.91 : Sauerstoff
                      78.33 % Stickstoff      0.00 %

KONDENSAT        :    10.06 cbm/h Propan     17.82 cbm/h i-Butan
                      84.73 cbm/h n-Butan    14.69 cbm/h i-Pent:
                      11.69 cbm/h n-Pentan   14.39 cbm/h Hexan -
                       1.80 cbm/h Benzol/Toluol
...
```

Tabelle 2

KREUZSTROMVERFAHREN MIT NACHFOLGENDER KONDENSATION MISCHBARER KOMPONENTEN

|  | FLUESSE LO | EXPONENTEN | DAMPFDRUECKE |
|---|---|---|---|
|  | IN cbm/qm*h*bar | IN 1/bar | IN bar |

| | | | | |
|---|---|---|---|---|
| Propan | : | 11.0000 | 0.1700 | 10.9040 |
| i-Butan | : | 9.9000 | 0.6700 | 3.7600 |
| n-Butan | : | 9.9000 | 0.6700 | 2.8000 |
| i-Pentan | : | 21.0000 | 0.8100 | 1.1000 |
| n-Pentan | : | 21.0000 | 0.8100 | 0.8230 |
| Hexan-Oktan | : | 20.0000 | 0.0000 | 0.2520 |
| Benzol-Toluol | : | 20.0000 | 0.0000 | 0.1610 |
| Sauerstoff | : | 1.3600 | 0.0000 | |
| Stickstoff | : | 0.6800 | 0.0000 | |
| | : | 0.0000 | 0.0000 | |

SCHRITTFLAECHE       :    0.5000 qm

MEMBRANFLAECHE   :   15.0000 qm      PERMEATDRUCK :     0.2000 bar
                                     DRUCKVERHAELTNIS :    50.00
                                     KONDENSATDRUCK :   10.0000 bar

ZULAUF          :   261.18 cbm/h
ZUSAMMENSETZUNG :     3.90 % Propan                6.89 % i-Butan
                     32.73 % n-Butan               5.63 % i-Pentan
                      4.48 % n-Pentan              5.51 % Hexan-Oktan
                      0.69 % Benzol-Toluol        10.24 : Sauerstoff
                     29.93 % Stickstoff            0.00 %

ZULAUF (GEMISCH) :   483.23 cbm/h
ZUSAMMENSETZUNG :      6.90 % Propan               6.65 % i-Butan
                      28.05 % n-Butan              3.74 % i-Pentan
                       2.84 % n-Pentan             3.14 % Hexan-Oktan
                       0.39 % Benzol-Toluol       18.38 : Sauerstoff
                      29.91 % Stickstoff           0.00 %

ZULAUF (GEMISCH) NACH
KONDENSATION    :   326.99 cbm/h
ZUSAMMENSETZUNG :      7.09 % Propan               4.33 % i-Butan
                      15.32 % n-Butan              1.04 % i-Pentan   .
                       0.62 % n-Pentan             0.23 % Hexan-Oktan
                       0.02 % Benzol-Toluol       27.16 : Sauerstoff
                      44.20 % Stickstoff           0.00 %

PERMEAT         :   222.05 cbm/h
ZUSAMMENSETZUNG :     10.43 % Propan               6.38 % i-Butan
                      22.55 % n-Butan              1.52 % i-Pentan
                       0.91 % n-Pentan             0.34 % Hexan-Oktan
                       0.03 % Benzol-Toluol       27.95 : Sauerstoff
                      29.89 % Stickstoff           0.00 %

RETENTAT        :   104.93 cbm/h
ZUSAMMENSETZUNG :      0.01 % Propan               0.00 % i-Butan
                       0.01 % n-Butan              0.00 % i-Pentan
                       0.00 % n-Pentan             0.00 % Hexan-Oktan
                       0.00 % Benzol-Toluol       25.48 : Sauerstoff
                      74.50 % Stickstoff           0.00 %

KONDENSAT       :     10.18 cbm/h Propan          17.99 cbm/h i-Butan
                      85.47 cbm/h n-Butan         14.70 cbm/h i-Pentan
                      11.70 cbm/h n-Pentan        14.39 cbm/h Hexan-Ok
                       1.80 cbm/h Benzol-Toluol
***

# Tabelle 3

```
FLUESSE IN cbm/qm*h*bar, EXPONENTEN IN 1/bar :

Propan        :  11.0000    0.1700    i-Butan       :    9.9000    0.67C
n-Butan       :   9.9000    0.6700    i-Pentan      :   21.0000    0.81C
n-Pentan      :  21.0000    0.8100    Hexan-Oktan   :   20.0000    0.00C
Benzol-Toluol :  20.0000    0.0000    Sauerstoff    :    1.3600    0.00C
Stickstoff    :   0.6800    0.0000                  :    0.0000    0.00C

SCHRITTFLAECHE      :    2.0000 qm

MEMBRANFLAECHE  :  80.0000 qm    PERMEATDRUCK  :   0.2000 bar
                                 DRUCKVERHAELTNIS :  10.00

ZULAUF          :  300.00 cbm/h
ZUSAMMENSETZUNG :    3.40 % Propan              6.00 % i-Butan
                    28.50 % n-Butan             4.90 % i-Pentan
                     3.90 % n-Pentan            4.80 % Hexan-Oktan
                     0.60 % Benzol-Toluol      10.10 : Sauerstoff
                    37.80 % Stickstoff          0.00 %

PERMEAT         :  261.44 cbm/h
ZUSAMMENSETZUNG :    3.90 % Propan              6.88 % i-Butan
                    32.70 % n-Butan             5.62 % i-Pentan
                     4.48 % n-Pentan            5.51 % Hexan-Oktan
                     0.69 % Benzol-Toluol      10.25 : Sauerstoff
                    29.98 % Stickstoff          0.00 %

RETENTAT        :   38.56 cbm/h
ZUSAMMENSETZUNG :    0.01 % Propan              0.01 % i-Butan
                     0.04 % n-Butan             0.00 % i-Pentan
                     0.00 % n-Pentan            0.00 % Hexan-Oktan
                     0.00 % Benzol-Toluol       9.11 : Sauerstoff
                    90.84 % Stickstoff          0.00 %
```

Tabelle 3

Bezugszeichenliste

| | |
|---|---|
| 10 | Luft/Permanentgasgemisch (Rohmedium) |
| 11 | erste Gastrennmembraneinrichtung |
| 12 | organisch angereicherter Gasstrom (Permeat) |
| 13 | organisch anbereicherter Gasstrom (Retentat) |
| 14 | Rückgewinnungseinrichtung |
| 15 | Verdichter |
| 16 | Vakuumpumpe |
| 17 | Abluft (Gasstrom) |
| 18 | Druckregler |
| 19 | zweite Gastrennmembraneinrichtung |
| 20 | abgereicherter Gasstrom von 19 |
| 21 | angereicherter Gasstrom von 19 |
| 22 | Druckregler |
| 23 | dritte Gastrennmembraneinrichtung |
| 24 | sauerstoffabgereicherter Gasstrom von 23 |
| 25 | sauerstoffangereicherter Gasstrom von 23 |
| 26 | Pumpeneinrichtung |
| 27 | Saugseite der Pumpeneinrichtung |
| 28 | Verdichter |
| 29 | Druckregler |
| 30 | Vorratsbehälter |

EP 0 329 962 B1

**Patentansprüche**

**1.** Verfahren zum Austrag organischer Verbindungen aus Luft/Permanentgasgemischen, bei dem das Luft/Permanentgasgemisch (Rohmedium) auf eine erste Gastrennmembraneinrichtung geleitet und in einen mit organischen Verbindungen angereicherten (Permeat) und in einen abgereicherten (Retentat) Gasstrom aufgetrennt wird, wobei permeatseitig ein unteratmosphärischer Druck erzeugt wird, und aus dem über eine Rückgewinnungseinrichtung geleiteten angereicherten Gasstrom die organischen Verbindungen zurückgewonnen werden, und der die Gastrennmembraneinrichtung verlassende abgereicherte Gasstrom an die Umgebung abgegeben wird, und daß ein aus der Rückgewinnungseinrichtung austretender Gasstrom erneut dem Luft/Permanentgasgemisch zugeführt wird, nachdem dieses druckerhöht wurde, dadurch gekennzeichnet, daß der Druck des primären Luft/Permanentgasgemisches vor Eintritt in die erste Gastrennmembraneinrichtung durch Verdichtung weit über Normaldruck erhöht wird und der Druck des angereicherten Gasstroms nach Austritt aus der Gastrennmembraneinrichtung zur Schaffung eines großen Druckverhältnisses weit unter den Druck des verdichteten Luft/Permanentgasgemisches erniedrigt wird, und der Druck des angereicherten Gasstroms vor Eintritt in die Rückgewinnungseinrichtung durch Verdichtung wiederum erhöht wird.

**2.** Verfahren nach Anspruch 1, dadurch gekennzeichnet, daß der die Rückgewinnungseinrichtung verlassende Gasstrom dem primären Luft/Gasgemisch unmittelbar vor Eintritt in die Gastrennmembran zugeführt wird.

**3.** Verfahren nach einem oder beiden der Ansprüche 1 oder 2, dadurch gekennzeichnet, daß die Abluft nach Austritt aus der Rückgewinnungseinrichtung in ihrem Druck vermindert wird.

**4.** Verfahren nach Anspruch 1, dadurch gekennzeichnet, daß die die Rückgewinnungseinrichtung verlassende Abluft auf eine zweite Gastrennmembraneinrichtung gegeben wird, deren abgereicherter Gasstrom an die Umgebung abgegeben wird und deren angereicherter Gasstrom unmittelbar mit dem die erste Gastrennmembraneinrichtung verlassenden angereicherten Gasstrom zusammengeführt wird.

**5.** Verfahren nach Anspruch 4, dadurch gekennzeichnet, daß zur Einstellung des Betriebsdrucks der zweiten Gastrennmembraneinrichtung der Druck des diese verlassenden Gasstroms einstellbar ist.

**6.** Verfahren nach einem oder mehreren der Ansprüche 1 bis 5, dadurch gekennzeichnet, daß das druckerhöhte primäre Luft/Permanentgasgemisch auf eine dritte Gastrennmembraneinrichtung gegeben wird, deren sauerstoffabgereicherter Gasstrom in die erste Gastrennmembraneinrichtung eintritt und deren sauerstoffangereicherter Gasstrom an die Umgebung abgegeben wird, wobei die dritte Gastrennmembraneinrichtung erhöhte sauerstoffpassierende Eigenschaften aufweist.

**7.** Verfahren nach einem oder mehreren der Ansprüche 1 bis 5, dadurch gekennzeichnet, daß der angereicherte Gasstrom vor Eintritt in die Rückgewinnungseinrichtung auf eine dritte Gastrennmembraneinrichtung gegeben wird, deren sauerstoffabgereicherter Gasstrom auf die Rückgewinnungseinrichtung und deren sauerstoffangereicherter Gasstrom an die Umgebung gegeben wird, wobei die dritte Gastrennmembraneinrichtung erhöhte sauerstoffpassierende Eigenschaften aufweist.

**8.** Verfahren nach einem oder mehreren der Ansprüche 1 bis 5, dadurch gekennzeichnet, daß die aus der Rückgewinnungseinrichtung austretende Abluft auf eine dritte Gastrennmembraneinrichtung gegeben wird, deren sauerstoffabgereicherter Gasstrom auf die zweite Gastrennmembraneinrichtung gegeben wird und deren sauerstoffangereicherter Gasstrom an die Umgebung abgegeben wird, wobei die dritte Gastrennmembraneinrichtung erhöhte sauerstoffpassierende Eigenschaften aufweist.

**9.** Verfahren nach einem oder beiden der Ansprüche 7 oder 8, dadurch gekennzeichnet, daß der aus der zweiten Gastrennmembraneinrichtung austretende abgereicherte Gasstrom vor Abgabe an die Umgebung zum Antrieb einer Pumpeneinrichtung genutzt wird, wobei der sauerstoffangereicherte Gasstrom der dritten Gastrennmembraneinrichtung auf die Saugseite der Pumpeneinrichtung zur Erzeugung eines Unterdrucks des Gasstroms gegeben wird.

**10.** Verfahren nach einem oder mehreren der Ansprüche 1 bis 9, dadurch gekennzeichnet, daß der abgereicherte Gasstrom nach Austritt aus der ersten Gastrennmembraneinrichtung in seinem Druck

11

EP 0 329 962 B1

vermindert wird.

**11.** Verfahren nach einem oder mehreren der Ansprüche 1 bis 10, dadurch gekennzeichnet, daß die Rückgewinnung der organischen Verbindungen aus dem angereicherten Gasstrom in die Rückgewinnungseinrichtung durch Wärmeentzug erfolgt.

**12.** Verfahren nach einem oder mehreren der Ansprüche 1 bis 11, dadurch gekennzeichnet, daß die Rückgewinnung der organischen Verbindungen aus dem angereicherten Gasstrom in der Rückgewinnungseinrichtung durch Kondensation unter Druck erfolgt.

**13.** Verfahren nach einem oder mehreren der Ansprüche 1 bis 12, dadurch gekennzeichnet, daß die Rückgewinnung der organischen Verbindungen aus dem angereicherten Gasstrom in der Rückgewinnungseinrichtung durch Sorption erfolgt.

**14.** Verfahren nach einem oder mehreren der Ansprüche 1 bis 13, dadurch gekennzeichnet, daß die Gastrennmembraneinrichtung eine Polyetherimid-Komposit-Membran gebildet ist.

**15.** Verfahren nach einem oder mehreren der Ansprüche 1 bis 14, dadurch gekennzeichnet, daß die Gastrennmembraneinrichtung eine asymmetrische Polyetherimidmembran ist.

**16.** Verfahren nach einem oder mehreren der Ansprüche 1 bis 15, dadurch gekennzeichnet, daß das zu trennende Luft/Permanentgasgemisch vor Druckerhöhung inertisiert wird.

**Claims**

**1.** A process for the discharge of organic compounds from mixtures of permanent gas and air, in which the air/permanent gas mixture (raw medium) is conducted over a first gas-separating membrane means and separated into one gas stream enriched with organic compounds (permeate) and another gas stream which is depleted (retentate), whereby on the permeate side a below-atmospheric pressure is generated and the organic compounds are recovered from the enriched flow of gas which is guided over a recovery means, the depleted gas stream leaving the gas-separating membrane means being discharged to the ambient, and in that a stream of gas emerging from the recovery means is again fed to the air/permanent gas mixture after which this latter is raised in pressure, characterised in that the pressure of the primary air/permanent gas mixture is increased to a level far above normal pressure by compression prior to its entering a first gas-separating membrane means and the pressure of the enriched gas stream, after leaving the gas-separating membrane means is lowered to far below the pressure of the compressed air/permanent gas mixture in order to create a considerable pressure ratio, and the pressure of the enriched gas stream is again increased by compression before it enters the recovery means.

**2.** A process according to Claim 1, characterised in that the gas stream leaving the recovery means is fed to the primary air/gas mixture immediately prior to entering the gas-separating membrane.

**3.** A process according to one or both of Claims 1 or 2, characterised in that after it leaves the recovery means, the pressure of the waste air is reduced.

**4.** A process according to Claim 1, characterised in that the waste air leaving the recovery means is passed to a second gas-separating membrane means, from which the depleted gas stream is delivered to the ambient while the enriched gas stream is combined directly with the enriched gas stream leaving the first gas-separating membrane means.

**5.** A process according to Claim 4, characterised in that in order to adjust the operating pressure of the second gas-separating membrane means, the pressure of the stream of gas leaving this is adjustable.

**6.** A process according to one or more of Claims 1 to 5, characterised in that the primary air/permanent gas mixture which is at increased pressure is passed to a third gas-separating membrane means, from which the oxygen-depleted gas stream enters the first gas-separating membrane means while the oxygen-enriched gas stream is delivered to the ambient, the third gas-separating membrane means

12

EP 0 329 962 B1

having enhanced oxygen-passing properties.

7.  A process according to one or more of Claims 1 to 5, characterised in that before it enters the recovery means the enriched stream of gas is passed over a third gas-separatng membrane means from which the oxygen-depleted stream of gas is passed to the recovery means while the oxygen-enriched stream of gas is passed to the ambient, the third gas-separating membrane means having enhanced oxygen-passing properties.

8.  A process according to one or more of Claims 1 to 5, characterised in that the waste air emerging from the recovery means is passed to a third gas-separating membrane means from which the oxygen-depleted gas stream is passed to the second gas-separating membrane means while the oxygen-enriched gas stream is passed to the ambient, the third gas-separating means having enhanced oxygen-passing properties.

9.  A process according to one or both of Claims 7 or 8, characterised in that before it is passed to the ambient, the depleted stream of gas emerging from the second gas-separating membrane means is used to drive a pump means, the oxygen-enriched stream of gas from the third gas-separating membrane means being passed to the intake side of the pump means in order to generate a negative pressure in the gas stream.

10.  A process according to one or more of Claims 1 to 9, characterised in that after the depleted stream of gas emerges from the first gas-separating membrane means, its pressure is reduced.

11.  A process according to one or more of Claims 1 to 10, characterised in that organic compounds are recovered from the enriched stream of gas in the recovery means by the extraction of heat.

12.  A process according to one or more of Claims 1 to 11, characterised in that organic compounds are recovered from the enriched stream of gas in the recovery means by condensation under pressure.

13.  A process according to one or more of Claims 1 to 12, characterised in that the recovery of organic compounds from the enriched stream of gas in the recovery means is carried out by sorption.

14.  A process according to one or more of Claims 1 to 13, characterised in that the gas-separating membrane means consists of a composite polyetherimide membrane.

15.  A process according to one or more of Claims 1 to 14, characterised in that the gas-separating membrane means is an asymmetric polyetherimide membrane.

16.  A process according to one or more of Claims 1 to 15, characterised in that prior to the pressure being raised, the mixture of air and permanent gas which is to be separated is rendered inert.

**Revendications**

1.  Procédé d'extraction de composés organiques à partir de mélanges gaz permanent/air, dans lesquels le mélange gazeux (milieu brut) est conduit dans un premier équipement à membrane de séparation gazeuse, et se trouve séparé en un courant gazeux enrichi en composés organiques (perméat) et en un courant gazeux appauvri (rétentat), dans lequel du côté du perméat est produite une pression inférieure à la pression atmosphérique, et à partir duquel les composés organiques sont récupérés à partir du courant gazeux enrichi provenant d'un équipement de récupération, le courant gazeux appauvri sortant de l'équipement à membrane à séparation gazeuse étant délivré à l'atmosphère, et dans lequel un courant gazeux provenant du dispositif de récupération est recyclé dans le mélange gaz permanent/air, après que l'on a élevé la pression de ce dernier, caractérisé en ce que la pression du mélange gazeux primaire est portée par compression au-dessus de la pression normale avant l'entrée dans le premier équipement à membrane à séparation gazeuse, en ce que la pression du courant gazeux enrichi est abaissée au-dessous de la pression du mélange gazeux compressé, après la sortie de ce courant gazeux de l'équipement à membrane de séparation gazeuse pour la réalisation d'un rapport de pression élevé, et en ce que la pression du courant gazeux enrichi est élevée par compression avant son entrée dans l'équipement de récupération.

13

EP 0 329 962 B1

**2.** Procédé selon la revendication 1, caractérisé en ce que le courant gazeux provenant de l'équipement de récupération, est recyclé au mélange gaz/air immédiatement avant l'entrée dans la membrane de séparation gazeuse.

**3.** Procédé selon une ou deux des revendications 1 ou 2, caractérisé en ce qu'on baisse la pression de l'air d'échappement après sa sortie de l'équipement de récupération.

**4.** Procédé selon la revendication 1, caractérisé en ce que l'air d'échappement provenant de l'équipement de récupération est délivré à un deuxième dispositif à membrane de séparation gazeuse dont le courant gazeux appauvri est délivré à l'atmosphère et dont le courant gazeux enrichi est immédiatement incorporé au courant gazeux enrichi provenant du premier équipement à membrane de séparation gazeuse.

**5.** Procédé selon la revendication 4, caractérisé en ce que pour régler la pression du deuxième dispositif à membrane de séparation gazeuse, la pression du courant gazeux quittant ce dernier est ajustable.

**6.** Procédé selon l'une ou plusieurs des revendications 1 à 5, caractérisé en ce que le mélange gaz/air primaire dont la pression a été élevée est délivré à un troisième dispositif à membrane de séparation gazeuse dont le courant gazeux appauvri en oxygène est dirigé dans le premier équipement à membrane de séparation gazeuse, et dont le courant gazeux enrichi en oxygène est délivré à l'atmosphère, le troisième dispositif à membrane de séparation gazeuse présentant des propriétés de perméabilité à l'oxygène plus élevées.

**7.** Procédé selon l'une ou plusieurs des revendications 1 à 5, caractérisé en ce que le courant gazeux enrichi est dirigé vers une troisième installation à membrane de séparation gazeuse avant d'être introduit dans l'équipement de récupération, le courant gazeux appauvri en oxygène étant dirigé vers l'équipement de récupération et le courant gazeux enrichi en oxygène étant rendu à l'atmosphère, la troisième installation à membrane de séparation gazeuse présentant des propriétés de perméabilité à l'oxygène plus élevées.

**8.** Procédé selon l'une ou plusieurs des revendications 1 à 5, caractérisé en ce que l'air d'échappement provenant de l'équipement de récupération est délivré à une troisième installation de membrane à séparation gazeuse, dont le courant gazeux appauvri en oxygène est délivré au deuxième dispositif de membrane à séparation gazeuse et dont le courant gazeux enrichi en oxygène est rendu à l'atmosphère, la troisième installation de membrane à séparation gazeuse présentant des propriétés de perméabilité à l'oxygène plus élevées.

**9.** Procédé selon l'une ou deux des revendications 7 ou 8, caractérisé en ce que le courant gazeux appauvri sortant du deuxième dispositif de membrane à séparation gazeuse est utilisé, avant d'être délivré dans l'atmosphère, pour l'entraînement d'un système de pompe, le courant gazeux enrichi en oxygène de la troisième installation de membrane à séparation gazeuse étant délivré au niveau d'un côté d'admission du dispositif de pompe pour mettre le courant gazeux sous pression.

**10.** Procédé selon l'une ou plusieurs revendications 1 à 9, caractérisé en ce que le courant gazeux appauvri subit une baisse de pression après sa sortie du premier équipement de membrane à séparation gazeuse.

**11.** Procédé selon l'une ou plusieurs des revendications 1 à 10, caractérisé en ce que la récupération des composés organiques à partir du courant gazeux enrichi, se produit dans le dispositif de récupération par l'intermédiaire d'une absorption de chaleur.

**12.** Procédé selon l'une ou plusieurs des revendications 1 à 11, caractérisé en ce que la récupération des composés organiques à partir du courant gazeux enrichi, se produit au niveau de l'équipement de récupération par condensation sous l'effet de la pression.

**13.** Procédé selon l'une ou plusieurs des revendications 1 à 12, caractérisé en ce que la récupération des composés organiques à partir du courant gazeux enrichi, se produit dans l'équipement de récupération par sorption.

14

**14.** Procédé selon l'une ou plusieurs des revendications 1 à 13, caractérisé en ce que l'équipement de membrane à séparation gazeuse est construit en membrane composites de polyétherimides.

**15.** Procédé selon l'une ou plusieurs des revendications 1 à 14, caractérisé en ce que l'équipement de membrane à séparation gazeuse est une membrane asymétrique de polyétherimides.

**16.** Procédé selon l'une ou plusieurs des revendications 1 à 15, caractérisé en ce que l'on rend inerte le mélange gaz/air avant d'en élever la pression.

EP 0 329 962 B1

# Fig. 1

# Fig. 2

16

# Fig. 3

# Fig. 4

# Fig. 5

# Fig. 6

Flux Density vs Temperature (P = 1230 mbar)

EP 0 329 962 B1

# Fig. 7

n - Butane Flux Density vs Pressure

20

# Fig. 8

Propane Flux Density vs Pressure

# Fig. 9

Gas fluxes through two types of polyethe-rimide / silicone rubber composite mem-branes